(19) Europäisches Patentamt
European Patent Office
Office européen des brevets

(11) **EP 3 677 313 A1**

(12) **EUROPEAN PATENT APPLICATION**

(43) Date of publication:
**08.07.2020  Bulletin 2020/28**

(51) Int Cl.:
***A62B 18/00*** (2006.01)

(21) Application number: **19150499.2**

(22) Date of filing: **07.01.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **KONG, Terry**
  **5656 AE Eindhoven (NL)**
• **CHEN, Weizhong**
  **5656 AE Eindhoven (NL)**
• **SU, William**
  **5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **A POLLUTION MASK AND CONTROL METHOD**

(57)    A pollution mask has a driven fan, wherein a rotation speed of the fan is monitored, as well as the external temperature and optionally also humidity levels. The fan rotation speed or change in fan rotation speed is used to obtain a first value which relates to a depth of breathing and a second value which relates to a rate of breathing. These are used, in combination with the ambient temperature and optionally also the ambient humidity level, to set the fan speed. Thus, the fan speed is set taking into account the breathing characteristics of the user as well as the ambient environmental conditions.

FIG. 2

**Description**

FIELD OF THE INVENTION

This invention relates to a pollution mask, for providing filtered air to the wearer of the mask, with the flow assisted by a fan.

BACKGROUND OF THE INVENTION

The World Health Organization (WHO) estimates that 4 million people die from air pollution every year. Part of this problem is the outdoor air quality in cities. The worst in class are Indian cities like Delhi that have an annual pollution level more than 10 times the recommended level. Well known is Beijing with an annual average 8.5 times the recommended safe levels. However, even in European cities like London, Paris and Berlin, the levels are higher than recommended by the WHO.

Since this problem will not improve significantly on a short time scale, the only way to deal with this problem is to wear a mask which provides cleaner air by filtration. To improve comfort and effectiveness one or two fans can be added to the mask. These fans are switched on during use and are typically used at a constant voltage. For efficiency and longevity reasons these are normally electrically commutated brushless DC fans.

The benefit to the wearer of using a powered mask is that the lungs are relieved of the slight strain caused by inhalation against the resistance of the filters in a conventional non-powered mask.

Furthermore, in a conventional non-powered mask, inhalation also causes a slight negative pressure within the mask which leads to leakage of the contaminants into the mask, which leakage could prove dangerous if these are toxic substances. A powered mask delivers a steady stream of air to the face and may for example provide a slight positive pressure, which may be determined by the resistance of an exhale valve, to ensure that any leakage is outward rather than inward.

There are several advantages if the fan operation or speed is regulated. This can be used to improve comfort by more appropriate ventilation during the inhalation and exhalation sequence or it can be used to improve the electrical efficiency. The latter translates into longer battery life or increased ventilation. Both of these aspects need improvement in current designs.

To regulate the fan speed, the pressure inside the mask can be measured and both pressure as well as pressure variation can be used to control the fan.

For example, the pressure inside a mask can be measured by a pressure sensor and the fan speed can be varied in dependence on the sensor measurements. A pressure sensor is costly so it would be desirable to provide an alternative method of monitoring pressure inside a mask.

The applicant has proposed, but not yet published, a solution in which a rotation speed of the fan is used as a proxy for pressure measurement. A pressure or a pressure change is determined based on the rotation speed of the fan. Using this pressure information, the breathing pattern of the user can be tracked it can also be determined whether the mask is worn or not. This approach is described in EP 17185742.8.

The applicant has also proposed, but not yet published, a solution in which the inner mask environment (temperature and relative humidity) is used as an indicator for use in controlling the fan speed. However, sensors in the mask chamber are susceptible to error due to condensation which may deteriorate the sensor response. Also, the response of a temperature and humidity sensor is generally slow so may not be able to track changes at the required rate. The change in temperature detected between breathing in and breathing out is also small in hot weather conditions.

Known control methods also do not easily take account of the activities of a user, such as their level of exertion.

There is therefore a need for a fan speed control method and apparatus which can be implemented at low cost and which can provide fan speed control which takes account of a user's activity level.

SUMMARY OF THE INVENTION

The invention is defined by the claims.

According to examples in accordance with an aspect of the invention, there is provided a pollution mask comprising:

an air chamber;
a filter which forms a boundary between the air chamber and the ambient surroundings outside the air chamber;
a fan for drawing air from outside the air chamber into the air chamber and/or drawing air from inside the air chamber to the outside;
a means for determining a rotation speed of the fan;

a temperature sensor for measuring an ambient temperature outside the air chamber; and
a controller which is adapted to:

derive from the determined fan rotation speed or change in fan rotation speed a first value which relates to a depth of breathing and a second value which relates to a rate of breathing; and
set a fan speed in dependence on the first value, the second value and the ambient temperature.

This mask provides automatic adjustment of the fan speed based on the fan rotation speed and the external temperature. By taking the external temperature into account, an estimate of user comfort is made. The fan speed adjustment is able to provide different air flows under different environmental conditions (temperature) and for different levels of activity of the user. Taking fast walking for example, users may need a large air flow to help to manage the climate in the mask during the summer, whereas relatively less air flow is needed in the winter in order to avoid making the wearer cold. The use of ambient temperature measurement, i.e. outside the mask chamber, avoids water condensation issues.

This provides a low cost solution, for example the required external sensing which can easily be integrated onto a PCB board.

The first and second values enable user activities to be taken into account. The first value for example relates to a depth of breathing, and by this is meant there is a positive correlation between the first value and the depth of breathing. More generally, the first value may for example relate to (i.e. correlate with) a magnitude of a pressure fluctuation across the fan. The pressure fluctuations are caused by breathing when the mask is worn and in normal use. The second value has a positive correlation with the rate of breathing. This may be used as another indicator of the activity level of the user, in addition to the depth of breathing as indicated by the first value.

The invention relates to a pollution mask. By this is meant a device which has the primary purpose of filtering ambient air to be breathed by the user. The mask does not perform any form of patient treatment. In particular, the pressure levels and flows resulting from the fan operation are intended solely to assist in providing comfort (by influencing the temperature or relative humidity in the air chamber) and/or to assist in providing a flow across a filter without requiring significant additional breathing effort by the user. The mask does not provide overall breathing assistance compared to a condition in which the user does not wear the mask.

In this system, fan speed monitoring is also used instead of having pressure measurement. To measure the fan speed, the fan itself may be used so that no additional sensors are required. The chamber may be closed in normal use, so that pressure fluctuations in the chamber have an influence on the load conditions of the fan and hence alter the fan electrical characteristics. Similarly, the fan electrical characteristics may determine the nature of the chamber, for example its volume, and if it is an open or closed volume.

The first value is for example based on a maximum swing in fan rotation speed during a sampling window. This swing is representative of the degree of pressure fluctuation, and hence it relates to the depth of breathing. The second value is for example a frequency based on the time between a consecutive maxima and minima in the fan rotation speed. This time period corresponds to half the breathing period, hence a frequency directly derived from this value corresponds to double the breathing rate (i.e. frequency).

The sampling window is chosen to be sufficient to capture at least one full breathing cycle, for example 6 seconds to capture a full breathing cycle at the lowest breathing rate of 10 breaths per minute.

The filter for example forms a boundary directly between the air chamber and the ambient surroundings outside the air chamber. This provides a compact arrangement which avoids the need for flow transport passageways. It means the user is able to breathe in through the filter. The filter may have multiple layers. For example, an outer layer may form the body of the mask (for example a fabric layer), and an inner layer may be for removing finer pollutants. The inner layer may then be removable for cleaning or replacement, but both layers may together be considered to constitute the filter, in that air is able to pass through the structure and the structure performs a filtering function.

The filter thus preferably comprises an outer wall of the air chamber and optionally one or more further filter layers. This provides a particularly compact arrangement and enables a large filter area, because the mask body performs the filtering function. The ambient air is thus provided directly to the user, when the user breathes in, through the filter.

For an exhaust fan, the pressure may be positive or negative (compared to the ambient pressure), for example if the fan is off there will be negative pressure caused by inhalation and a positive pressure caused by exhalation. If an exhaust fan is on, negative or positive pressures are possible during breathing depending on the fan speed and breathing characteristics. A negative pressure will result during periods of no breathing.

If the fan is for providing an increased pressure in the air chamber (e.g. a flow into the air chamber during inhalation), it is only required to provide a small increased pressure, for example for assisting inhalation of the user.

In all cases, a maximum pressure in the air chamber in use is for example below 4 cmH$_2$O, for example below 2 cmH$_2$O, for example below 1 cmH$_2$O, higher than the pressure outside the air chamber.

The mask may further comprise a humidity sensor for measuring an ambient humidity level outside the air chamber, wherein the controller is adapted to set a fan speed further in dependence on the humidity level. Thus, both the external ambient temperature and humidity may be taken into account. This provides increased accuracy in the determination of the environmental conditions.

For example, the controller may be adapted to:

derive from the ambient temperature and the ambient humidity level a heat index value which relates to a measure of comfort.

This heat index value is representative of the general ambient environmental conditions and can be used so that the fan speed is set taking into account the expected user comfort in those particular conditions.

The heat index value may comprise a polynomial function of the ambient temperature, the ambient humidity level and one or more powers of the ambient temperature and/or ambient humidity level. For example, it may comprise a function of the ambient temperature, the square of the ambient temperature, the ambient humidity level and the square of the ambient humidity level.

The controller may be adapted to:

generate from the current fan rotation speed and the first and second values an instruction to increase the fan speed, decrease the fan speed or keep the fan speed the same.

Thus, the depth and rate of breathing are used to control whether a change in fan speed is needed, taking into account the current fan speed.

The controller may be adapted to:

determine from the ambient temperature and the ambient humidity level an amount by which the fan speed should be increased or decreased.

Thus, the environmental conditions determine the amount by which a fan speed adjustment is needed.

The controller may be adapted to determine from the fan rotation speed or change in fan rotation speed that the mask is not worn to turn off the fan if it is determined that the mask is not worn.

In order to detect if the mask is worn, the fan rotation signal may be analyzed. This may take account both of the first value, which is indicative of the depth of breathing (when breathing is detected), as well as the second value, which is indicative of the breathing rate (when breathing is detected).

By determining if the mask is worn, the mask design enables power to be saved when the mask is not being worn, but without requiring any additional sensors. In particular, if there is no detected pressure differential across the mask, this indicates that both sides are at atmospheric pressure and the mask is not being worn. In effect, there is no longer a closed or partially closed chamber, so that the air chamber is open to the atmosphere. The fan may be turned off if it is detected that the mask is not worn.

The fan may be driven by an electronically commutated brushless motor, and the means for determining rotation speed comprises an internal sensor of the motor. Alternatively, the means for determining rotation speed may comprise a circuit for detecting a ripple on the electrical supply to a motor which drives the fan.

An internal sensor is already provided in such motors to enable rotation of the motor. The motor may even have an output port on which the internal sensor output is provided. Thus, there is a port which carries a signal suitable for determining the rotation speed. Alternatively, a ripple is detected which results from switching current through the motor coils, which cause induced changes in the supply voltage as a result of the finite impedance of the input voltage source.

The fan may be a two-wire fan and the circuit for detecting a ripple comprises a high pass filter. The additional circuitry needed for a motor which does not already have a suitable fan speed output can be kept to a minimum.

The controller may be adapted to:

determine a respiration cycle from the fan rotation speed or change in fan rotation speed; and

to control an outlet valve in dependence on the phase of the respiration cycle and/or to turn off the fan during an inhalation time.

The rotation monitoring thus provides a simple way to determine inhalation phases, which may then be used to control the timing of a venting valve of the mask or to determine whether or not the mask is worn and hence in use.

The controller may be adapted to turn off the fan during an inhalation time. This may be used to save power. Shutting down the fan during inhalation may be desirable for a user who does not have difficulty breathing through the filter, to save power if configured in such a way.

The fan may be only for drawing air from inside the air chamber to the outside. In this way, it may at the same promote a supply of fresh filtered air to the air chamber even during exhalation, which improves user comfort. In this case, the pressure in the air chamber may be below the outside (atmospheric) pressure at all times so that fresh air is always supplied to the face.

The outlet valve may comprise a passive pressure-regulated check valve or an actively driven electrically controllable valve. This may be used to make the mask more comfortable. During inhalation, by closing the valve (actively or passively), it is prevented that unfiltered air is drawn in. During exhalation, the valve is opened so that breathed out air is expelled.

The invention also provides a non-therapeutic method of controlling a pollution mask, wherein the pollution mask is not a mask for delivering therapy to a patient, the method comprising:

drawing gas into and/or out of an air chamber of the mask using a fan which forms a boundary between the air chamber and the ambient surroundings outside the air chamber;
determining a rotation speed of the fan;
deriving from the determined fan rotation speed or change in fan rotation speed a first value which relates to a depth of breathing and a second value which relates to a rate of breathing;
measuring an ambient temperature outside the air chamber; and
setting a fan speed in dependence on the first value, the second value and the ambient temperature.

The method may additionally comprise measuring an ambient humidity level outside the air chamber, and setting a fan speed is further in dependence on the humidity level. A heat index value may be derived from the temperature and humidity measurements, which relates to a measure of comfort.

The method may comprise:

generating from the current fan rotation speed and the first value an instruction to increase the fan speed, decrease the fan speed or keep the fan speed the same; and
determine from the ambient temperature and the ambient humidity level an amount by which the fan speed should be increased or decreased.

BRIEF DESCRIPTION OF THE DRAWINGS

Examples of the invention will now be described in detail with reference to the accompanying drawings, in which:

Figure 1 shows a pressure monitoring system implemented as part of a face mask;
Figure 2 shows one example of the components of the pressure monitoring system;
Figure 3 shows an example of a combined temperature and humidity sensor;
Figure 4A shows a rotation signal during inhalation and during exhalation and Figure 4B shows how a fan rotation speed varies over time; and
Figure 5 shows a circuit for controlling the current through one of the stators of a brushless DC motor;
Figure 6 is used to explain a heat index measure; and
Figure 7 shows a mask operating method.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a pollution mask having a driven fan, wherein a rotation speed of the fan is monitored, as well as external temperature and optionally also humidity levels. The fan rotation speed or change in fan rotation speed is used to obtain a first value which relates to a depth of breathing and a second value which relates to a rate of breathing. These are used, in combination with the ambient temperature and optionally the ambient humidity level, to set the fan speed. Thus, the fan speed is set taking into account the breathing characteristics of the user as well as the ambient environmental conditions.

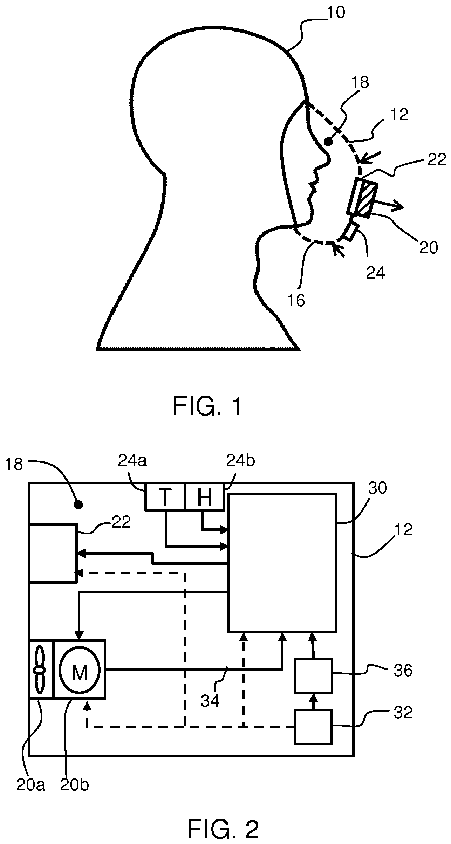

Figure 1 shows a face mask with automatic fan speed control.

A subject 10 is shown wearing a face mask 12 which covers the nose and mouth of the subject. The purpose of the mask is to filter air before it is breathed in the subject. For this purpose, the mask body itself acts as an air filter 16. Air is drawn in to an air chamber 18 formed by the mask by inhalation. During inhalation, an outlet valve 22 such as a check valve is closed due to the low pressure in the air chamber 18.

A sensing arrangement 24 is provided for measuring at least the ambient temperature, and preferably both the ambient temperature and humidity (e.g. relative or absolute humidity) outside the mask chamber 18.

The filter 16 may be formed only by the body of the mask, or else there may be multiple layers. For example, the mask body may comprise an external cover formed from a porous textile material, which functions as a pre-filter. Inside the external cover, a finer filter layer is reversibly attached to the external cover. The finer filter layer may then be removed for cleaning and replacement, whereas the external cover may for example be cleaned by wiping. The external cover also performs a filtering function, for example protecting the finer filter from large debris (e.g. mud), whereas the finer filter performs the filtering of fine particulate matter. There may be more than two layers. Together, the multiple layers function as the overall filter of the mask.

When the subject breathes out, air is exhausted through the outlet valve 22. This valve is opened to enable easy exhalation, but is closed during inhalation. A fan 20 assists in the removal of air through the outlet valve 22. Preferably, more air is removed than exhaled so that additional air is supplied to the face. This increases comfort due to lowering relative humidity and cooling. During inhalation, by closing the valve, it is prevented that unfiltered air is drawn in. The timing of the outlet valve 22 is thus dependent on the breathing cycle of the subject. The outlet valve may be a simple passive check valve operated by the pressure difference across the filter 16. However, it may instead be an electronically controlled valve.

There will be a varied pressure inside the chamber if the mask is worn and the user is breathing. In particular the chamber is closed by the face of the user. The pressure inside the closed chamber when the mask is worn will also vary as a function of the breathing cycle of the subject. When the subject breathes out, there will be a slight pressure increase and when the subject breathes in there will be a slight pressure reduction.

If the fan is driven with a constant drive level (i.e. voltage), the different prevailing pressure will manifest itself as a different load to the fan, since there is a different pressure drop across the fan. This altered load will then result in a different fan speed. The rotation speed of the fan may thus be used as a proxy for a measurement of pressure across the fan.

For a known pressure (e.g. atmospheric pressure) at one side of the fan, the pressure monitoring enables determination of a pressure, or at least a pressure change, on the other side of the fan. This other side is for example a closed chamber which thus has a pressure different to atmospheric pressure.

The pressure variation, as detected based on monitoring the fan rotation speed, may be used to obtain information about the breathing of the user. In particular, a first value may represent the depth of breathing and a second value may represent the rate of breathing. According to the invention, the first and second values, as well as the ambient temperature and the ambient humidity level, are used to set the fan speed. Furthermore, by detecting an equal pressure on each side the fan (or other conditions relating to the first and second values), it can also be determined that the chamber is not closed but is connected to atmospheric pressure on both sides.

This will result in a variation of the fan speed which falls below a threshold. This situation may thus be used to determine that the mask is not worn and hence not in use. This information can be used to switch off the fan to save power.

The means for determining a rotation speed may comprise an already existing output signal from the fan motor or a separate simple sensing circuit may be provided as an additional part of the fan. However, in either case the fan itself is used so that no additional sensors are required.

Figure 2 shows one example of the components of the system. The same components as in Figure 1 are given the same reference numbers. The sensing arrangement is shown as a separate temperature sensor 24a and humidity sensor 24b.

In addition to the components shown in Figure 1, Figure 2 shows a controller 30, a local battery 32 and a means 36 for determining the fan rotation speed.

The fan 20 comprises a fan blade 20a and a fan motor 20b. In one example, the fan motor 20b is an electronically commutated brushless motor, and the means for determining rotation speed comprises an internal sensor of the motor. Electronically commutated brushless DC fans have internal sensors that measure the position of the rotor and switch the current through the coils in such a way that the rotor rotates. The internal sensor is thus already provided in such motors to enable feedback control of the motor speed.

The motor may have an output port on which the internal sensor output 34 is provided. Thus, there is a port which carries a signal suitable for determining the rotation speed.

Alternatively, the means for determining the rotation speed may comprise a circuit 36 for detecting a ripple on the electrical supply to the motor 20b. The ripple results from switching current through the motor coils, which cause induced changes in the supply voltage as a result of the finite impedance on the battery 32. The circuit 36 for example comprises a high pass filter so that only the signals in the frequency band of the fan rotation are processed. This provides an extremely simple additional circuit, and of much lower cost than a conventional pressure sensor.

This means the motor can be of any design, including a two-wire fan with no in-built sensor output terminal. It will also work with a DC motor with brushes.

If the outlet valve 22 is an electronically switched value, the respiration cycle timing information may then be used to control the outlet valve 22 in dependence on the phase of the respiration cycle. The fan speed monitoring thus provides a simple way to determine inhalation phases, which may then be used to control the timing of the outlet valve 22 of the mask.

In addition to controlling the outlet valve, the controller may turn off the fan during an inhalation time or an exhalation time. This gives the mask different operating modes, which may be used to save power.

For a given drive level (i.e. voltage) the fan speed increases at lower pressure across the fan because of the reduced load on the fan blades. This gives rise to an increased flow. Thus, there is an inverse relationship between the fan speed and the pressure difference.

This inverse relationship may be obtained during a calibration process or it may be provided by the fan manufacturer. The calibration process for example involves analyzing the fan speed information over a period during which the subject is instructed to inhale and exhale regularly with normal breathing. The captured fan speed information can then be matched to the breathing cycle, from which threshold values can then be set for discriminating between inhalation and exhalation.

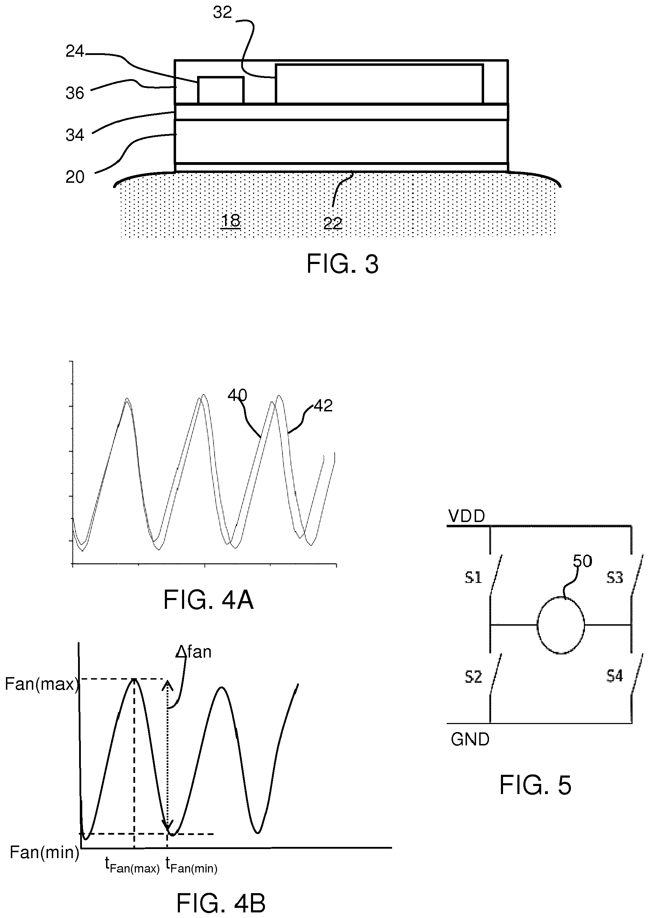

Figure 3 shows one possible design of a module incorporating the fan, check valve and the sensing arrangement 24. The module comprises a printed circuit board 34 which carries the battery 32 and sensor arrangement 24 on one side, and the fan 20 and valve 22 is mounted on the other side. A cover 36 is provided over the top side. The sensing arrangement faces outside the mask.

Figure 4A shows schematically the rotor position (as a measured sensor voltage) against time.

The rotational speed may be measured from the frequency of the AC component (caused by the switching events in the motor) of the DC voltage to the fan. This AC component originates from the current variation that the fan draws, imposed on the impedance of the power supply.

Figure 4A shows the signal during inhalation as plot 40 and during exhalation as plot 42. There is a frequency reduction during exhalation caused by an increased load on the fan by the increased pressure gradient. The observed frequency changes thus results from the different fan performance during the breathing cycle.

Figure 4B shows the frequency variation over time, by plotting the fan rotation speed versus time. There is a maximum difference in fan rotation speed $\Delta$fan between successive maxima and minima, and this correlates with the depth of breathing. This is the first value derived from the fan rotation signal. The time between these points is used to derive the second value, for example the frequency corresponding to this time period (which is then twice the breathing rate).

Note that the first value maybe obtained from the raw fan rotation signal or there may be smoothing carried out first. Thus, there are at least two different two ways to calculate the maximum swing, based on untreated real-time speeds or treated speeds. In practice, there is noise or other fluctuations added on the real-time signals. A smoothing algorithm may be used to treat the real-time signal and calculate the first value from the smoothed signal.

During the exhalation, fan operation forces air out of the area between face and mask. This enhances comfort because exhalation is made easier. It can also draw additional air onto the face which lowers the temperature and relative humidity. Between inhalation and exhalation, the fan operation increases comfort because fresh air is sucked into the space between the face and the mask thereby cooling that space.

During inhalation, the outlet valve is closed (either actively or passively) and the fan can be switched off to save power. This provides a mode of operation which is based on detecting the respiration cycle.

The precise timing of the inhalation and exhalation phases can be inferred from previous respiration cycles, if the fan is turned off for parts of the respiration cycle, and hence not giving pressure information.

For the fan assisted exhalation, power needs to be restored just before the exit valve opens again. This also makes sure that the next inhale-exhale cycle remains properly timed and sufficient pressure and flow are made available.

Around 30% power savings are easily achievable using this approach, resulting in prolonged battery life. Alternatively, the power to the fan can be increased by 30% for enhanced effectiveness.

With different fan and valve configurations the measurement of the fan rotation speed enables control to achieve increased comfort.

In fan configurations where the filter is in series with the fan the pressure monitoring may be used to measure the flow resistance of the filter, in particular based on the pressure drop across the fan and filter. This can be done at switch on, when the mask is not on the face for a period of time. That resistance can be used as a proxy for the age of the filter.

As mentioned above, a fan using an electronically commutated brushless DC motor has internal sensors that measure the position of the rotor and switch the current through the coils in such a way that the rotor rotates.

Figure 5 shows an H-bridge circuit which functions as an inverter to generate an alternating voltage to the stator coils 50 of the motor from a DC supply VDD, GND. The inverter has a set of switches S1 to S4 to generate an alternating voltage across the coil 50. The switches are controlled by signals which depend on the rotor position, and these rotor position signals may be used to monitor the fan rotation.

As mentioned above, the system preferably measures both ambient temperature and humidity. These two measurements may be combined to provide a measure of a comfort level, such as a measure known as a heat index.

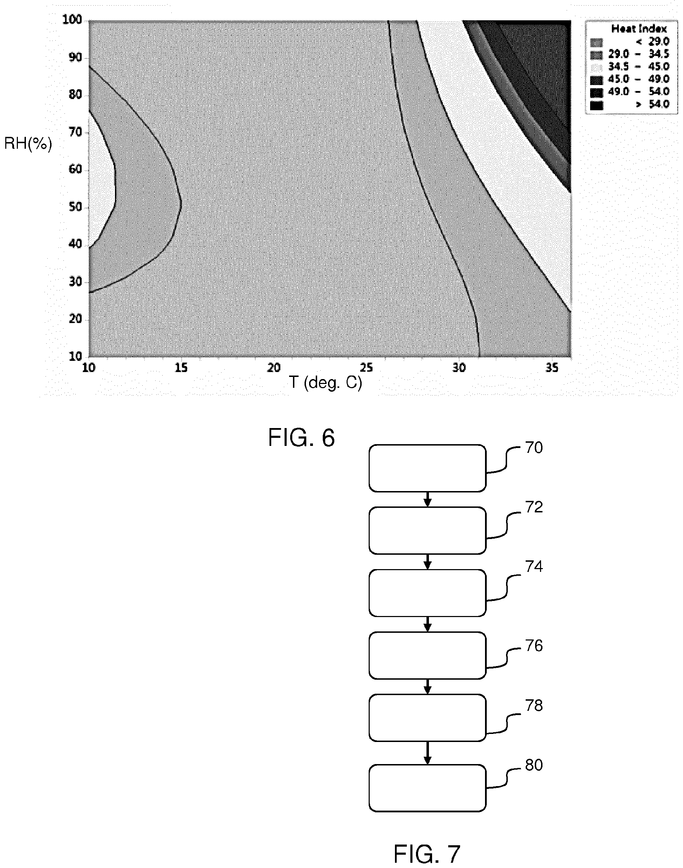

Figure 6 shows a chart of relative humidity (%RH) versus temperature (degrees Celsius). Different regions are shaded differently to show different heat index values.

The heat index is a function of the ambient temperature (T), the square of the ambient temperature, the ambient humidity level (rh) and the square of the ambient humidity level. An example is:

$$\begin{aligned} Index_{heat} = {} & -42.379 + (2.04901523 \times T) + (10.14333127 \times rh) \\ & - (0.22475541 \times T \times rh) - (6.83783 \times 10^{-3} \times T^2) \\ & - (5.481717 \times 10^{-2} \times rh^2) + (1.22874 \times 10^{-3} \times T^2 \times rh) \\ & + (8.5282 \times 10^{-4} \times T \times rh^2) - (1.99 \times 10^{-6} \times T^2 \times rh^2) \end{aligned}$$

The heat index gives a measure of the degree of comfort under different temperature and relative humidity conditions. For example, less than 29 is categorized as no discomfort, 29 to 34.5 is categorized as acceptable, 34.5 to 39 is categorized as some discomfort, 39 to 45 is categorized as great discomfort, 45 to 54 is categorized as dangerous and over 54 corresponds to imminent heat stroke. Thus, the higher the index value, the more discomfort is felt. The heat index is applicable when T>10 °C. For T<10 °C other chilling parameters should be taken into account.

The controller sets the initial fan speed rotation using the Table 1 below:

Table 1

| Heat Index | <29 | 29-34.5 | 34.5-39 | 39-45 | >45 |
|---|---|---|---|---|---|
| T>10 deg. | 4000rpm | 5000rpm | 6000rpm | 7000rpm | 8000rpm |
| T<10 deg. | 4000rpm | | | | |

When T>10 °C, the initial fan rotation speed increases with an increase of heat index. While T<10 °C, a relatively low fan rotation is provided. The low fan rotation makes sure that user doesn't feel too cold.

The rotation speeds in the table are of course just examples.

The fan rotation signals are obtained in real-time and the first value, representing the depth of breathing, and the second value, representing the depth of breathing, are continuously monitored. The normal breathing rate for an adult at rest is 12-18 bpm (breath per minute), and it increases when people are performing activities, e.g., running, fast walking or bicycling.

The sampling window should contain at least 1 breathing cycle, otherwise the breathing rate cannot be acquired.

The first value, representing the depth of breathing, may be based on the maximum swing in rotation speed captured within a sampling window (of at least one breath, for example 5 seconds) as shown in Figure 4B:

$$\Delta fan = Fan(max) - Fan(min)$$

The second value, representing the rate of breathing, may be given by the frequency corresponding to the timing difference for those maximum and minimum values:

$$f = 1/\left| t_{Fan(max)} - t_{Fan(min)} \right|$$

The second value is thus a frequency based on the time between a consecutive maxima and minima in the fan rotation speed. Again, the second value may be obtained from the raw fan rotation signal or there may be smoothing carried out first.

A breathing rate may be defined as 0.5f. For the calculation of f, another method is to identify the inflection points from descending (ascending) to ascending (descending).

For the real-time adjustment of the fan rotation speed, one example makes use of a lookup Table 2 as shown below.

There are four sub-tables below. Each one is for a different range of breathing rates (i.e. different ranges for the second value) and each one shows how the breathing depth (i.e. the first value $\Delta fan$) alters how the fan speed needs to be changed.

Tables 2

| Fan_level | Base rotation | 8≤ f <15 bpm | | | | | |
|---|---|---|---|---|---|---|---|
| | | Δfan 100-350 (rpm) | Δfan 350-550 (rpm) | Δfan 550-650 (rpm) | Δfan 650-1000 (rpm) | Δfan 1000-1400 (rpm) | Δfan >1400 (rpm) |
| 1 | 5500 (49%) | - | Fan+ | Fan+ | Fan++ | Fan++ | Fan++ |
| 2 | 6500 (60%) | Fan- | - | Fan+ | Fan+ | Fan++ | Fan++ |
| 3 | 7400 (74%) | Fan- | Fan- | Fan- | - | Fan+ | Fan++ |
| 4 | 8000 (84%) | Fan-- | Fan-- | Fan- | Fan- | - | Fan+ |
| 5 | 8500 (89%) | Fan-- | Fan-- | Fan- | Fan- | - | - |

| Fan_level | Base rotation | 15≤ f < 21 bpm | | | | | |
|---|---|---|---|---|---|---|---|
| | | Δfan 100-350 (rpm) | Δfan 350-550 (rpm) | Δfan 550-650 (rpm) | Δfan 650-1000 (rpm) | Δfan 1000-1400 (rpm) | Δfan >1400 (rpm) |
| 1 | 5500 (49%) | - | Fan+ | Fan+ | Fan++ | Fan++ | Fan++ |
| 2 | 6500 (60%) | Fan- | - | Fan+ | Fan+ | Fan++ | Fan++ |
| 3 | 7400 (74%) | Fan- | Fan- | Fan- | - | Fan+ | Fan++ |
| 4 | 8000 (84%) | Fan-- | Fan-- | Fan- | Fan- | - | Fan+ |
| 5 | 8500 (89%) | Fan-- | Fan-- | Fan- | Fan- | - | - |

| Fan_level | Base rotation | 21 ≤ f < 30 bpm | | | | | |
|---|---|---|---|---|---|---|---|
| | | Δfan 100-350 (rpm) | Δfan 350-550 (rpm) | Δfan 550-650 (rpm) | Δfan 650-1000 (rpm) | Δfan 1000-1400 (rpm) | Δfan >1400 (rpm) |
| 1 | 5500 (49%) | - | Fan+ | Fan+ | Fan++ | Fan++ | Fan++ |
| 2 | 6500 (60%) | Fan- | - | Fan+ | Fan+ | Fan++ | Fan++ |
| 3 | 7400 (74%) | Fan- | Fan- | Fan- | - | Fan+ | Fan++ |
| 4 | 8000 (84%) | Fan-- | Fan-- | Fan- | Fan- | - | Fan+ |
| 5 | 8500 (89%) | Fan-- | Fan-- | Fan- | Fan- | - | - |

| Fan_level | Base rotation | f: ≥ 30 bpm | | | | | |
|---|---|---|---|---|---|---|---|
| | | Δfan 100-350 (rpm) | Δfan 350-550 (rpm) | Δfan 550-650 (rpm) | Δfan 650-1000 (rpm) | Δfan 1000-1400 (rpm) | Δfan >1400 (rpm) |
| 1 | 5500 (49%) | - | Fan+ | Fan+ | Fan++ | Fan++ | Fan++ |
| 2 | 6500 (60%) | Fan- | - | Fan+ | Fan+ | Fan++ | Fan++ |
| 3 | 7400 (74%) | Fan-- | Fan- | Fan- | - | Fan+ | Fan++ |
| 4 | 8000 (84%) | Fan-- | Fan-- | Fan- | Fan- | - | Fan+ |
| 5 | 8500 (89%) | Fan-- | Fan-- | Fan- | Fan- | - | - |

In each sub-table, "-" means the fan speed is to keep running at the current level, "Fan+, Fan-, Fan++ and Fan--" denote how to change the fan speed.

It can be seen that the desired change to the fan speed depends on the current level of the fan speed (so it tends towards the desired value as indicated by "-"), on the breathing rate (in breaths per minute) and on the breathing depth Δfan.

The meaning of Fan+, Fan-, Fan++ and Fan-- is made clear by Table 3 below.

Table 3

| Heat Index | <34.5 | 34.5-45 | >45 |
|---|---|---|---|
| Fan+ | n=1 | n=2 | n=3 |
| Fan++ | n=2 | n=3 | n=3 |
| Fan- | n=2 | n=1 | n=1 |
| Fan-- | n=3 | n=2 | n=1 |

The value n represents how many step changes in fan speed are made as between the fan speed settings 1 to 5 in the tables above.

For example if the current fan level is Level 1 and if n =3 (such as for the scenario: "Fan+" AND heat index>45), then the fan should be changed to Level 4 (1+3).

For the initial fan speed determination, when the fan is turned on by the user, a suitable initial fan speed is needed, not always from the lowest speed. Since the heat index can generally reflect the comfort level of user under different environmental conditions, the heat index is used to set the initial speed as shown above.

When adapting the fan speed in real time, the breathing rates and volumes change with user's activities. For example, normal sitting with a breathing rate of 12 bpm and 0.5 L tidal volume will probably result in <350 rpm fan speed change (Δfan) when the fan level is -5500 rpm (see above Table 2). If the activity increases, e.g., from sitting to walking (e.g., 20 bpm, 1 L volume), the Δfan will be increased above 350 rpm, which requires an increased fan level. The heat index is used to help to determine which level the fan should be changed to.

The tables above provide only one detailed implementation, and of course different versions are possible for different fan designs etc.

The mask may be for covering only the nose and mouth (as shown in Figure 1) or it may be a full face mask. The mask is for filtering ambient air.

The mask design described above has the main air chamber formed by the filter material, through which the user breathes in air.

An alternative mask design has the filter in series with the fan as also mentioned above. In this case, the fan assists the user in drawing in air through the filter, thus reducing the breathing effort for the user. An outlet valve enables breathed out air to be expelled and an inlet valve may be provided at the inlet.

The invention may again be applied for detecting the pressure variations caused by breathing for controlling the inlet valve and/or the outlet valve. The fan in this example needs to be turned on during inhalation, to assist the user in drawing air through the series filter, but it may be turned off during exhalation when the outlet valve is open. Thus, the pressure information derived may again be used to control the fan to save power when the fan operation is not needed. The detection of whether the mask is worn or not may also be implemented.

It will be seen that the invention may be applied to many different mask designs, with fan-assisted inhalation or exhalation, and with an air chamber formed by a filter membrane or with a sealed hermetic air chamber.

One option as discussed above is thus the use of the fan only for drawing air from inside the air chamber to the outside, for example when an exhaust valve is open. In such a case, the pressure inside the mask volume may be maintained by the fan below the external atmospheric pressure so that there is a net flow of clean filtered air into the mask volume during exhalation. Thus, low pressure may be caused by the fan by during exhalation and by the user during inhalation (when the fan may be turned off).

An alternative option is the use of the fan only for drawing air from the ambient surroundings to inside the air chamber. In such a case, the fan operates to increase the pressure in the air chamber, but the maximum pressure in the air chamber in use remains below 4 $cmH_2O$ higher than the pressure outside the air chamber, in particular because no high pressure assisted breathing is intended. Thus, a low power fan may be used.

In all cases, the pressure inside the air chamber preferably remains below 2 $cmH_2O$, or even below 1 $cmH_2O$ or even below 0.5 $cmH_2O$, above the external atmospheric pressure. The pollution mask is thus not for use in providing a continuous positive airway pressure, and is not a mask for delivering therapy to a patient.

The mask is preferably battery operated so the low power operation is of particular interest.

Figure 7 shows a mask operating method.

The method comprises:

in step 70, drawing gas into and/or out of an air chamber of the mask using the fan;
in step 72 determining a rotation speed of the fan;
in step 74, deriving from the determined fan rotation speed or a change in fan rotation speed a first value which relates to a depth of breathing and a second value which relates to a rate of breathing;
in step 76, measuring an ambient temperature outside the air chamber; and an ambient humidity level outside the air chamber;
in step 78, setting a fan speed in dependence on the first value, the second value, the ambient temperature and the ambient humidity level.

The method optionally includes determining that the mask is not worn in step 80 and then turning off the fan, based on the first value or the first and second values.

As discussed above, embodiments make use of a controller, which can be implemented in numerous ways, with software and/or hardware, to perform the various functions required. A processor is one example of a controller which employs one or more microprocessors that maybe programmed using software (e.g., microcode) to perform the required functions. A controller may however be implemented with or without employing a processor, and also maybe implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media maybe fixed within a processor or controller or may be transportable, such that the one or more programs stored thereon can be loaded into a processor or controller.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

**Claims**

1. A pollution mask comprising:

an air chamber (18);
a filter (16) which forms a boundary between the air chamber and the ambient surroundings outside the air chamber;
a fan (20) for drawing air from outside the air chamber (18) into the air chamber and/or drawing air from inside the air chamber to the outside;
a means (34, 36) for determining a rotation speed of the fan;
a temperature sensor (24a) for measuring an ambient temperature outside the air chamber; and
a controller (30) which is adapted to:

derive from the determined fan rotation speed or change in fan rotation speed a first value which relates to a depth of breathing and a second value which relates to a rate of breathing; and
set a fan speed in dependence on the first value, the second value and the ambient temperature.

2. A mask as claimed in claim 1, wherein the first value is based on a maximum swing in fan rotation speed during a sampling window.

3. A mask as claimed in claim 1 or 2, wherein the second value is a frequency based on the time between a consecutive maxima and minima in the fan rotation speed.

4. A mask as claimed in any one of claims 1 to 3, further comprising a humidity sensor (24b) for measuring an ambient humidity level outside the air chamber, wherein the controller is adapted to set a fan speed further in dependence on the humidity level.

5. A mask as claimed in claim 4, wherein the controller is adapted to:

derive from the ambient temperature and the ambient humidity level a heat index value which relates to a measure of comfort.

6. A mask as claimed in claim 5, wherein the heat index value comprises a polynomial function of the ambient temperature, the ambient humidity level and one or more powers of the ambient temperature and/or ambient humidity level.

7. A mask as claimed in any one of claims 4 to 6, wherein the controller is adapted to:

generate from the current fan rotation speed and the first and second values an instruction to increase the fan speed, decrease the fan speed or keep the fan speed the same.

8. A mask as claimed in claim 7, wherein the controller is adapted to:

determine from the ambient temperature and the ambient humidity level an amount by which the fan speed should be increased or decreased.

9. A mask as claimed in any one of claims 1 to 8, wherein the controller is adapted determine from the fan rotation speed or change in fan rotation speed that the mask is not worn to turn off the fan if it is determined that the mask is not worn.

10. A mask as claimed in any one of claims 1 to 9, wherein:

the fan (20) is driven by an electronically commutated brushless motor, and the means for determining rotation speed comprises an internal sensor of the motor; or
the means (36) for determining rotation speed comprises a circuit for detecting a ripple on the electrical supply to a motor which drives the fan.

11. A mask as claimed in any one of claims 1 to 10, wherein the controller (30) is adapted to:

determine a respiration cycle from the fan rotation speed or change in fan rotation speed; and
to control an outlet valve (22) in dependence on the phase of the respiration cycle and/or to turn off the fan during an inhalation time.

**12.** A non-therapeutic method of controlling a pollution mask, wherein the pollution mask is not a mask for delivering therapy to a patient, the method comprising:

drawing gas into and/or out of an air chamber of the mask using a fan which forms a boundary between the air chamber and the ambient surroundings outside the air chamber;
determining a rotation speed of the fan;
deriving from the determined fan rotation speed or change in fan rotation speed a first value which relates to a depth of breathing and a second value which relates to a rate of breathing;
measuring an ambient temperature outside the air chamber; and
setting a fan speed in dependence on the first value, the second value and the ambient temperature.

**13.** A method as claimed in claim 12, comprising measuring an ambient humidity level outside the air chamber and wherein setting a fan speed is further in dependence on the humidity level.

**14.** A method as claimed in claim 13, comprising deriving from the ambient temperature and the ambient humidity level a heat index value which relates to a measure of comfort.

**15.** A method as claimed in claim 13 or 14, comprising:

generating from the current fan rotation speed and the first value an instruction to increase the fan speed, decrease the fan speed or keep the fan speed the same; and
determining from the ambient temperature and the ambient humidity level an amount by which the fan speed should be increased or decreased.

FIG. 1

FIG. 2

FIG. 3

FIG. 4A

FIG. 4B

FIG. 5

FIG. 6

FIG. 7

Europäisches
Patentamt

European
Patent Office

Office européen
des brevets

## EUROPEAN SEARCH REPORT

Application Number

EP 19 15 0499

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2016/157159 A1 (MICROSFERE PTE LTD [SG]) 6 October 2016 (2016-10-06)<br>* figures *<br>* page 45, line 16 - page 46, line 8 *<br>* page 19, lines 11-22 *<br>* page 27, line 19 - page 28, line 12 *<br>* page 55, line 14 - page 59, line 16 *<br>* claim 67 *<br>----- | 1-15 | INV.<br>A62B18/00 |
| A | GB 2 472 592 A (3M INNOVATIVE PROPERTIES CO [US]) 16 February 2011 (2011-02-16)<br>* claims 1,4 *<br>----- | 1,12 | |
| A,D | WO 2018/215225 A1 (KONINKLIJKE PHILIPS NV [NL]) 29 November 2018 (2018-11-29)<br>* claim 1 *<br>----- | 9 | |
| | | | TECHNICAL FIELDS SEARCHED (IPC)<br><br>A62B |

The present search report has been drawn up for all claims

| Place of search | Date of completion of the search | Examiner |
|---|---|---|
| The Hague | 13 June 2019 | Andlauer, Dominique |

EPO FORM 1503 03.82 (P04C01)

## ANNEX TO THE EUROPEAN SEARCH REPORT
## ON EUROPEAN PATENT APPLICATION NO.

EP 19 15 0499

This annex lists the patent family members relating to the patent documents cited in the above-mentioned European search report.
The members are as contained in the European Patent Office EDP file on
The European Patent Office is in no way liable for these particulars which are merely given for the purpose of information.

13-06-2019

| Patent document cited in search report | | Publication date | Patent family member(s) | | Publication date |
|---|---|---|---|---|---|
| WO 2016157159 | A1 | 06-10-2016 | CN | 107405508 A | 28-11-2017 |
| | | | EP | 3277386 A1 | 07-02-2018 |
| | | | JP | 2018512518 A | 17-05-2018 |
| | | | KR | 20170132188 A | 01-12-2017 |
| | | | SG | 11201707644Q A | 30-10-2017 |
| | | | TW | 201701915 A | 16-01-2017 |
| | | | US | 2018078798 A1 | 22-03-2018 |
| | | | WO | 2016157159 A1 | 06-10-2016 |
| GB 2472592 | A | 16-02-2011 | AU | 2010282612 A1 | 23-02-2012 |
| | | | CN | 102470260 A | 23-05-2012 |
| | | | EP | 2464429 A1 | 20-06-2012 |
| | | | GB | 2472592 A | 16-02-2011 |
| | | | JP | 5723366 B2 | 27-05-2015 |
| | | | JP | 2013501585 A | 17-01-2013 |
| | | | KR | 20120051735 A | 22-05-2012 |
| | | | US | 2012138051 A1 | 07-06-2012 |
| | | | WO | 2011019778 A1 | 17-02-2011 |
| WO 2018215225 | A1 | 29-11-2018 | CN | 108939337 A | 07-12-2018 |
| | | | WO | 2018215225 A1 | 29-11-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 17185742 A